# EUROPEAN PATENT SPECIFICATION

(11) **EP 1 238 628 B1**
(45) Date of publication and mention of the grant of the patent: **09.05.2007**
(21) Application number: 01610023.2
(22) Date of filing: 09.03.2001
(51) Int. Cl.: A61B 5/0484, A61B 5/0478

(54) **Device for determining acoustically evoked cerebral potentials**
Vorrichtung zur bestimmung akustisch evozierter Gehirnpotentiale
Dispositif pour determiner des potentiels cérebraux acoustiquement provoqués

(43) Date of publication of application: 11.09.2002
(73) Proprietor: Maico Diagnostic GmbH, 13629 Berlin (DE)
(72) Inventor: Köpke, Wolfgang, 13629 Berlin (DE)
(74) Representative: Kuhnen & Wacker

(56) References cited:
- EP-A- 0 813 840
- US-A- 3 998 213
- US-A- 5 601 091
- US-A- 5 606 978
- US-A- 5 755 230
- US-A- 5 813 404
- US-A- 5 954 667

## Description

### BACKGROUND OF THE INVENTION

The derivation of acoustically evoked electrical brain potentials of a subject is a known audiometric diagnostic method for testing hearing and for evaluating various causes of hearing damage without the active participation of the subject.

This method is referred to in the field as ERA (electric response audiometry) or BERA (brainstem electric response audiometry) or brainstem audiometry. Areas of application for this method include for example the performance of the first hearing tests in newborns, testing the hearing of infants or of unconscious persons such as accident victims for example, and the diagnosis of neurologic diseases, for example neurinomas of the acoustic nerve. Intraoperative hearing tests are also possible with this method.

Electrical brain potentials are triggered by acoustic stimulation of the ear with conduction through air or bone. Headphones are usually used for the purpose. The electrical signals that are thus generated by the brainstem are picked up by electrodes applied to the head. Usually three electrodes are used, namely one electrode to determine the reference potential and two active electrodes to derive the acoustically-evoked electrical signals at two different locations on the head.

Acoustic stimulation of the ear can take for example the form of click stimuli or, for direct determination of the hearing threshold, of a rapid sequence of clicks with increasing volume. Other types of stimuli are of course also possible. The brainstem generates potential waves at each click, which are averaged after being picked up and conducted away by the electrodes.

In the previously known devices the measuring results are relying on a constantly good electrical connection between the head and the electrode, resulting in low impedance.

The prior art document us 3998213 discloses a device for positioning electroencephalographic (EEG) electrodes. In the description col. 7, line 54-60 it is stated that a plurality of lights (LED's) may be provided for indicating when proper electrical contact has been established. This means that the LED s may be used for indicating operational readiness prior to actual operation.

The prior art document us 5954667 discloses an Acoustically Evoked Brain Potential measuring device having the possibility of indicating the readiness of the device. us 5954667 does however not indicate the obtaining of correct and usefull measurements. The device may be completely ready and functional and still the data obtained may be without any relevance or usefuilness for the further analysis due to the lack of a proper contact to the skin. The readiness is a status that is checked before the measurement is initiated.

In practise this is however nor always a simple task. The measurements may often be repeated due to poor measuring results, which are not detected during measurement. There is for this reason a need for improvement of the previously known devices of this type.

It is therefore an object of the invention to provide a device to permit simpler and easier use and hence achievement of better results while performing brainstem audiometry.

### SUMMARY OF THE INVENTION

This object is achieved by the device described in claim 1.

By providing a visual indication of the impedance level there is a possibility of checking this during measurement and hence provide an immediate correction to the positioning of the electrode so as to achieve the desired low impedance.

The means for visually indicating a level of impedance are located on the structure holding the electrodes, or a preamplifier where the electrodes are connected via cables. This allows for a simultaneous visual contact with the structure during a correction operation and the indicator means.

The means for determining the impedance comprises switching arrangements for each electrode enabling the switching of each electrode into a impedance measuring mode and back to normal brain potential measuring mode. This provides for a possibility of obtaining a reliable measurement of the impedance without disturbing the measurement.

Preferably the means for determining the impedance are integrated with the structure or the device. This also improves the operation and visibility of the indicator means.

In one preferred embodiment the means for visually indicating a level of impedance comprises a single light emitter, e.g. a diode, indicating either a low or a high impedance level.

In another preferred embodiment the means for visually indicating a level of impedance comprises two light emitters, e.g. diodes, where one is indicating a low impedance level and the other is indicating a high impedance level.

In an device according to the invention, the electrode unit can also include an electroencephalograph ("EEG") amplifier as a component, so that a minimum conduction path is provided between the pickup electrodes and the EEG amplifier, and thus the possibility of stray potentials being picked up is minimized.

### BRIEF DESCRIPTION OF THE DRAWINGS

FIG. 1 shows a schematic circuit forming part of the device according to the invention,
FIG. 2 shows a top view of a part of an example of a device according to the invention,
FIG. 3 shows a bottom view of a part of a device according to the invention,
FIG. 4 shows a side view of a part of a device according to the invention.

### DETAILED DESCRIPTION OF THE INVENTION

The device according to the invention will now be described briefly in terms of its important details, with reference to the embodiments shown in the enclosed drawings.

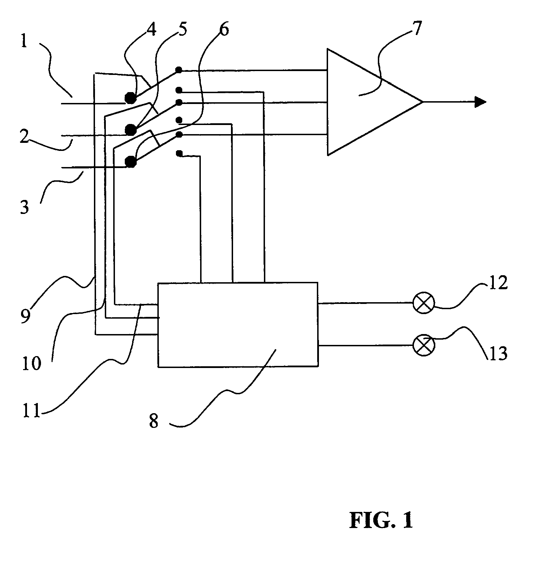

From FIG. 1 a schematic diagram appears. The diagram shows three inputs 1,2,3 each leading to a switch 4,5,6. The switches are switching between a measuring mode and a test mode. In the measuring mode the input signals are transmitted to an EEG amplifier 7 and further to an audiometer used in a measuring process. In the test mode the impedance is measured between the electrode and the skin of the individual on which the electrodes are placed. The test of the impedance is carried out with a predetermined sampling frequency and is controlled by the control electronics 8 adapted to control the switches via connections 9,10,11. The result of the impedance measurement is indicated by the diodes 12,13, where one indicated a too high level of the impedance and the other one indicated a satisfactory low level of the impedance.

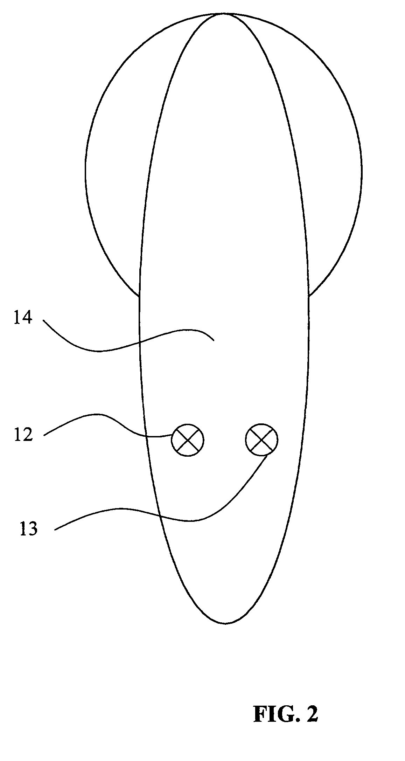

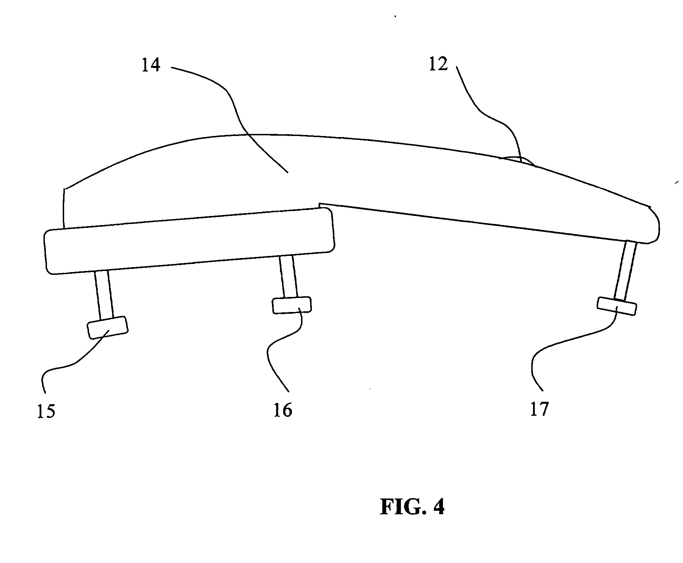

The device according to FIG. 2, 3 and 4 consists of a housing 14 with a plurality of arms that have electrodes 15,16,17 at their ends, a earphone 18 integrated in the housing. An EEG amplifier (not shown) forms part of the device.

A cable (not shown) connects the device with the rest of the audiometer used for brainstem audiometry, said audiometer generating the signals for acoustic stimulation of the ear and processing and evaluating the derived brainstem potentials. In the embodiment, a single cable is shown that can contain both a line to supply electrical click signals and also a line to conduct the preamplified brainstem potentials from EEG amplifier. Of course, separate cables or wireless transmission pathways can also be used for the purpose.

The electrical potentials generated in the brainstem by acoustic stimulation of the ear are picked up by the electrodes on the arms. Usually three electrodes are used, namely a reference electrode for detecting a reference potential and two pickup electrodes. The reference electrode is brought into contact with the head in front of the ear, and one of the two deriving electrodes is placed behind the ear and the other in the area of the crown of the head.

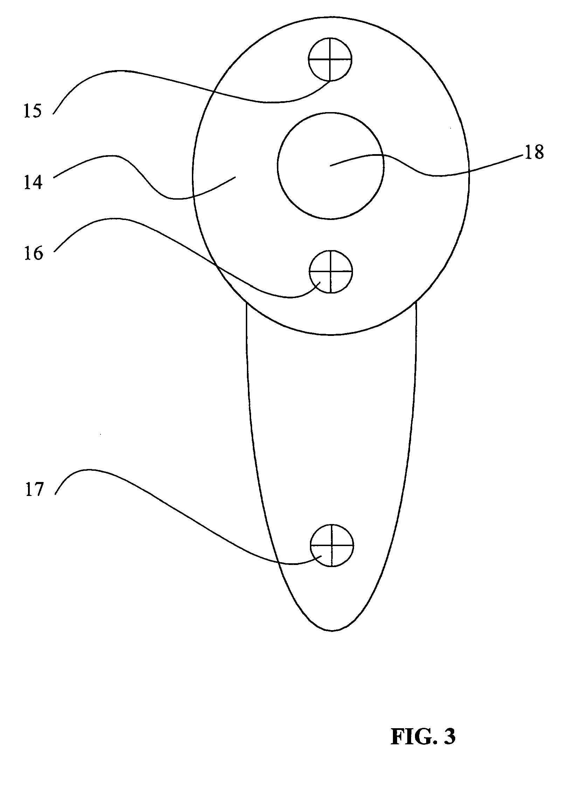

In the embodiment according to FIG. 3, electrode 17 is the pickup electrode that detects brainstem potentials in the vicinity of the crown of the head and electrode 16 is the reference electrode. The second pickup electrode 15 is applied to the head behind the ear.

## Claims

1. A device for use in determining acoustically evoked brain potentials in brainstem audiometry from electrodes applied to the head of a subject, the device comprising: a plurality of electrodes (15, 16, 17) comprising at least one pickup electrode and a reference electrode, to be applied at different points of the head, where the device comprises means (8) for determining the impedance between the electrodes and the head as well as means (12, 13) for visually indicating a level of impedance, where the electrodes are mounted on a structure (14) and where the means for visually indicating a level of impedance are located on the structure holding the electrodes **characterised in that** the means for determining the impedance comprises switching arrangements (4, 5, 6) for each electrode enabling the switching of each electrode into a impedance measuring mode and back to normal brain potential measuring mode.

2. A device according to claim 1, where the means for visually indicating a level of impedance are located on a preamplifier where the electrodes are connected via cables.

3. A device according to claim 1 or 2, where the means for determining the impedance are integrated with the device, preferably a structure holding the electrodes.

4. A device according to any of the preceding claims, where the means for visually indicating a level of impedance comprises a single light emitter, e.g a diode, indicating either a low or a high impedance level.

5. A device according to any of the preceding claims, where the means for visually indicating a level of impedance comprises two light emitters, e.g. diodes, where one is indicating a low impedance level and the other is indicating a high impedance level.

6. A device according to any of the preceding claims, where the means for information the level of the impedance is send via a cable ore wireless to the audiometer.

## Patentansprüche

1. Vorrichtung zur Verwendung beim Bestimmen akustisch evozierter Gehimpotentiale bei der Hirnstamm-Audiometrie von an den Kopf einer Person angelegten Elektroden, wobei die Vorrichtung umfasst: eine Mehrzahl von Elektroden (15, 16, 17), welche zumindest eine Aufnahmeelektrode und eine Referenzelektrode umfasst, zum Anlegen an verschiedenen Punkten des Kopfes, wobei die Vorrichtung ein Mittel (8) zum Bestimmen der Impedanz zwischen den Elektroden und dem Kopf sowie ein Mittel (12, 13) zum optischen Anzeigen eines Impedanzpegels aufweist, wobei die Elektroden auf eine Struktur (14) montiert sind und wobei das Mittel zum optischen Anzeigen eines Impedanzpegels auf der die Elektroden haltenden Struktur angeordnet ist,
**dadurch gekennzeichnet, dass**
das Mittel zum Bestimmen der Impedanz Schaltereinrichtungen (4, 5, 6) für jede Elektrode umfasst, welche das Schalten jeder Elektrode in einen Impedanzmessmodus und zurück in einen normalen Hirnpotentialmessmodus ermöglichen.

2. Vorrichtungen nach Anspruch 1, wobei das Mittel zum optischen Anzeigen eines Impedanzpegels auf einem Vorverstärker angeordnet ist, an den die Elektroden über Kabel angeschlossen sind.

3. Vorrichtung nach Anspruch 1 oder 2, wobei das Mittel zum Bestimmen der Impedanz in der Vorrichtung, vorzugsweise in einer die Elektroden haltenden Struktur, integriert ist.

4. Vorrichtung nach irgendeinem der vorhergehenden Ansprüche, wobei das Mittel zum optischen Anzeigen eines Impedanzpegels einen einzelnen Lichtsender, z. B. eine Diode, welche entweder einen geringen oder einen hohen Impedanzpegel anzeigt, umfasst.

5. Vorrichtung nach irgendeinem der vorhergehenden Ansprüche, wobei das Mittel zum optischen Anzeigen eines Impedanzpegels zwei Lichtsender, z. B. Dioden, umfasst, wobei einer einen geringen Impedanzpegel anzeigt und der andere einen hohen Impedanzpegel anzeigt.

6. Vorrichtung nach irgendeinem der vorhergehenden Ansprüche, wobei das Mittel für Information der Impedanzpegel über ein Kabel oder drahtlos an das Audiometer gesendet wird.

## Revendications

1. Dispositif à utiliser pour déterminer des potentiels cérébraux provoqués par stimulation acoustique en audiométrie du tronc cérébral, au moyen d'électrodes appliquées sur la tête d'un sujet, le dispositif comprenant une pluralité d'électrodes (15, 16, 17), dont au moins une électrode de détection et une électrode de référence, destinées à être appliquées sur des endroits différents de la tête, le dispositif comprenant des moyens (8) pour déterminer l'impédance entre les électrodes et la tête, ainsi que des moyens (12, 13) pour indiquer visuellement un niveau d'impédance, les électrodes étant montées sur une structure (14) et les moyens d'indication visuelle d'un niveau d'impédance étant situés sur la structure portant les électrodes, **caractérisé en ce que** les moyens de détermination de l'impédance comprennent des agencements de commutation (4, 5, 6) pour chaque électrode, permettant la commutation de chaque électrode vers un mode de mesure d'impédance puis son retour vers un mode de mesure de potentiel cérébral normal.

2. Dispositif selon la revendication 1, dans lequel les moyens d'indication visuelle d'un niveau d'impédance sont placés sur un préamplificateur auquel les électrodes sont connectées via des câbles.

3. Dispositif selon la revendication 1 ou 2, dans lequel les moyens de détermination de l'impédance sont intégrés au dispositif, de préférence une structure portant les électrodes.

4. Dispositif selon l'une quelconque des revendications précédentes, dans lequel les moyens d'indication visuelle d'un niveau d'impédance comprennent un unique émetteur de lumière, par exemple une diode, indiquant soit un niveau d'impédance bas soit un niveau d'impédance haut.

5. Dispositif selon l'une quelconque des revendications précédentes, dans lequel les moyens d'indication visuelle d'un niveau d'impédance comprennent deux émetteurs de lumière, par exemple des diodes, l'un indiquant un niveau d'impédance bas et l'autre indiquant un niveau d'impédance haut.

6. Dispositif selon l'une quelconque des revendications précédentes, dans lequel les moyens d'information du niveau d'impédance sont envoyés via un câble ou via une liaison sans fil à l'audiomètre.
